# EUROPÄISCHE PATENTSCHRIFT

(11) **EP 2 736 557 B1**
(45) Veröffentlichungstag und Bekanntmachung des Hinweises auf die Patenterteilung: **18.09.2019**
(21) Anmeldenummer: 12750542.8
(22) Anmeldetag: 27.07.2012
(51) Int. Cl.: A61M 1/16

(54) **ELEKTRONISCH GESTEUERTE GASMISCHEINHEIT ZUM ZUFÜHREN EINES SPÜLGASES ZU EINEM OXYGENERATOR**
ELECTRONICALLY CONTROLLED GAS MIXING UNIT FOR SUPPLYING A SWEEP GAS TO AN OXYGENERATOR
UNITÉ DE MÉLANGE DE GAZ À COMMANDE ÉLECTRONIQUE CONÇUE POUR AMENER UN GAZ DE PURIFICATION DANS UN GÉNÉRATEUR D'OXYGÈNE

(30) Priorität: 27.07.2011 DE 102011052189
(43) Veröffentlichungstag der Anmeldung: 04.06.2014
(73) Patentinhaber: Joost, Thilo, 61169 Friedberg (DE)
(72) Erfinder: JOOST, Thilo, 61169 Friedberg (DE); KÖBRICH, Rainer, 68809 Neulußheim (DE)
(74) Vertreter: Sattler de Sousa e Brito, Clara
(86) Internationale Anmeldenummer: PCT/EP2012/064802
(87) Internationale Veröffentlichungsnummer: WO 2013/014277

(56) Entgegenhaltungen:
- WO-A1-02/43792
- WO-A1-2005/092417
- WO-A2-03/092776
- US-A- 3 927 981
- US-A1- 2006 162 725
- US-A1- 2009 081 079

## Beschreibung

Die Erfindung betrifft eine Anordnung zum Entfernen von Kohlenstoffdioxid aus einem exkorporalen Blutstrom und/oder zum Anreichern des exkorporalen Blutstroms mit Sauerstoff. Die Anordnung umfasst einen Filter, der eine Membran hat, die einen Blutbereich von einem Gasbereich trennt. Durch den Blutbereich ist der exkorporale Blutstrom und durch den Gasbereich ein Gasstrom eines Spülgases geführt. Ferner hat die Anordnung einen ersten Gasvorratsbehälter, in dem ein erstes Gas aufgenommen ist, und mindestens einen zweiten Gasvorratsbehälter, in dem ein von dem ersten Gas verschiedenes zweites Gas aufgenommen ist. Darüber hinaus umfasst die Anordnung eine Gasmischeinheit zum Mischen des ersten und des zweiten Gases zu einem Spülgas, das über eine Zuführleitung dem Gasbereich des Filters zuführbar ist.

In der Medizin werden bei erkrankten Patienten sogenannte Oxygeneratoren verwendet, um aus dem Blut dieser Patienten Kohlenstoffdioxid zu entfernen und bei Indikation das Blut mit Sauerstoff anzureichern. Heutzutage werden fast ausschließlich Oxygeneratoren benutzt, die eine Membran umfassen, über die ein Blutbereich von einem Gasbereich getrennt ist. Das Blut wird den Patienten aus einem Hauptgefäß entnommen und vorzugsweise mittels einer Blutpumpe oder durch den Blutdruck des Patienten in den Blutbereich des Oxygenerators befördert. Durch den Gasbereich wird zeitgleich ein Spülgas transportiert, wobei als Spülgas in der Regel entweder reiner Sauerstoff oder ein Gemisch aus Sauerstoff und Stickstoff verwendet wird. Insbesondere wird ein Gemisch aus 21% Sauerstoff und 79% Stickstoff, sogenannte AIR, verwendet. Je nach Erkrankung des Patienten können auch weitere von den zuvor genannten Gasen abweichende Gase als Spülgas verwendet werden. Das Spülgas wird insbesondere aus in medizinischen Einrichtungen vorhandenen Wandentnahmestellen entnommen und entspricht somit den Anforderungen medizinischer Gase.

Durch den Druckgradienten des Partialdrucks bzw. durch den Konzentrationsgradienten des Kohlenstoffdioxid an der Membran wird Kohlenstoffdioxid von dem Blutbereich durch die Membran hindurch in den Gasbereich transportiert, wohingegen durch den Druckgradienten des Partialdrucks des Sauerstoffs bzw. durch den entsprechenden Konzentrationsgradienten des Sauerstoffs dieser durch die Membranen hindurch von dem Gasbereich in den Blutbereich transportiert wird, sodass das Blut mit Sauerstoff angereichert wird und gleichzeitig Kohlenstoffdioxid aus dem Blut entfernt wird. Die Menge an Kohlenstoffdioxid, die pro Zeiteinheit dem Blut entnommen wird und/oder die Menge an Sauerstoff, mit der das Blut pro Zeiteinheit angereichert wird, hängen u.a. von der Strömungsgeschwindigkeit des Spülgases durch den Gasbereich, der Strömungsgeschwindigkeit des Blutes durch den Blutbereich sowie von der Zusammensetzung des Spülgases ab.

Bei bekannten Anordnungen sind in der Regel zwei Gasvorratsbehälter vorgesehen, in denen unterschiedliche Gase aufgenommen sind. Über einfache mechanische Gasmischeinheiten, sogenannte Gasblender, kann das Spülgas aus diesen Gasen zusammengemischt werden. Insbesondere sind hierfür Stellenrädchen vorgesehen, die manuell betätigt werden können, wobei über die Stellrädchen die Öffnung eines Ventils der Gasvorratsbehälter verstellt wird und somit die Zusammensetzung durch die Menge des dem Gasvorratsbehälter entnommenen Gases verändert wird.

Problematisch an solchen bekannten Gasblendern ist es, dass mit ihrer Hilfe nur sehr ungenau die Zusammensetzung des Spülgases und/oder die Strömungsgeschwindigkeit des Spülgases, mit der dieser den Gasbereich des Oxygenerators durchströmt, eingestellt werden kann. Ferner ist dieses manuelle Einstellen aufwändig und fehleranfällig.

Es ist Aufgabe der Erfindung, eine Anordnung zum Entfernen von Kohlenstoffdioxid aus einem exkorporalen Blutstrom und/oder zum Anreichern des exkorporalen Blutstroms mit Sauerstoff anzugeben, bei der die Zusammensetzung eines Spülgases auf einfache Weise genau einstellbar ist.

Diese Aufgabe wird durch eine Anordnung mit dem Merkmal des Anspruchs 1 gelöst. Vorteilhafte Weiterbildungen der Erfindung sind in den abhängigen Ansprüchen angegeben.

Erfindungsgemäß umfasst die Anordnung eine elektronische Steuereinheit zum Steuern der Gasmischeinheit, die eine Eingabeeinheit umfasst, mit deren Hilfe der Anteil des ersten Gases an dem Spülgas durch eine Bedienperson einstellbar ist. Die Steuereinheit steuert die Gasmischeinheit derart an, dass das Spülgas den eingestellten Anteil des ersten Gases aufweist. Durch eine solche elektronisch gesteuerte Gasmischeinheit wird zum einen eine einfache komfortable Bedienung erreicht und zum anderen eine genaue Einstellbarkeit der Zusammensetzung des Spülgases gewährleistet. Insbesondere muss die Bedienperson nicht mehr selbst berechnen, wie weit welches Ventil welches Gasvorratsbehälters aufgedreht werden muss, um die gewünschte Strömungsgeschwindigkeit des Spülgases und die gewünschte Zusammensetzung des Spülgases zu erreichen.

Der Filter umfasst insbesondere einen Oxygenerator, über den das Entfernern des Kohlenstoffdioxids bzw. das Zuführen des Sauerstoffs auf einfache Weise möglich ist. Der Blutstrom wird insbesondere mit Hilfe einer Blutpumpe durch den Blutbereich des Filters befördert. Ferner kann auch eine Gasfördereinheit zum Erzeugen des Gasstroms durch den Gasbereich vorgesehen sein. Alternativ kann der Gasstrom des Spülgases auch lediglich durch den Druck, mit dem die Gase in den Gasvorratsbehältern aufgenommen sind, erzeugt werden.

Die Gasmischeinheit umfasst insbesondere ein erstes Proportionalventil zum Einstellen des aus dem ersten Gasvorratsbehälter entnommenen Volumenstroms des ersten Gases und eine zweites Proportionalventil zum Einstellen des aus dem zweiten Gasvorratsbehälter entnommenen Volumenstroms des zweiten Gases. Die Steuereinheit steuert das erste Proportionalventil und/oder das zweite Proportionalventil derart an, dass das Spülgas den gewünschten Anteil des ersten Gases und somit auch den gewünschten Anteil des zweiten Gases umfasst.

Die Steuereinheit ermittelt insbesondere in Abhängigkeit des eingestellten Anteils des ersten Gases einen ersten Stellwert für das erste Proportionalventil und/oder einen zweiten Stellwert für das zweite Proportionalventil und stellt diesen ersten Stellwert an dem ersten Proportionalventil und/oder den zweiten Stellwert einen zweiten Proportionalventil ein. Somit müssen die Stellwerte nicht aufwendig von der Bedienperson ermittelt oder ausprobiert werden, sondern es ist ein vollautomatisches Einstellen der gewünschten Zusammensetzung des Spülgases durch die elektronische Steuereinheit möglich.

Bei einer besonders bevorzugten Ausführungsform der Erfindung sind ein dritter Gasvorratsbehälter, in dem ein drittes Gas aufgenommen ist, und mindestens ein vierter Gasvorratsbehälter, in dem ein viertes Gas aufgenommen ist, vorgesehen. Hierdurch wird erreicht, dass die Anzahl der verschiedenen zu Verfügung stehenden Gase erhöht ist, sodass das verwendete Spülgas besonders genau an den Zustand des Patienten angepasst werden kann, ohne dass hierfür die Gasvorratsbehälter ausgetauscht werden müssen. Die Gasmischeinheit mischt das Spülgas hierzu aus dem ersten Gas, dem zweiten Gas, dem dritten Gas und/oder dem vierten Gas, wobei über die Eingabeeinheit der Anteil mindestens dreier der vier Gase einstellbar ist und die Steuereinheit die Gasmischeinheit derart ansteuert, dass das Spülgas die eingestellten Anteil der Gase aufweist.

Es ist besonders vorteilhaft, wenn die Gasmischeinheit ein drittes Proportionalventil zum Einstellen des aus dem dritten Gasvorratsbehälter entnommenen Volumenstroms des dritten Gases und/oder ein viertes Proportionalventil zum Einstellen des aus dem vierten Gasvorratsbehälter entnommenen Volumenstroms des vierten Gases umfasst. Die Steuereinheit steuert zum Einstellen des über die Eingabeeinheit eingestellten Anteils des dritten Gases das dritte Proportionalventil oder das vierte Proportionalventil entsprechend an. Insbesondere ermittelt die Steuereinheit wiederum in Abhängigkeit des eingestellten Anteils des dritten Gases einen dritten Stellwert für das dritte Proportionalventil und/oder in Abhängigkeit des eingestellten Anteils des vierten Gases einen vierten Stellwert für das vierte Proportionalventil und stellt an dem dritten bzw. vierten Proportionalventil den entsprechenden Stellwert ein.

Über die Eingabeeinheit ist insbesondere der Anteil aller vier Gase jeweils einstellbar, sodass die Bedienperson, unabhängig davon, welchen Gasanteil sie einstellen will, diesen direkt eingeben kann und nicht ersten einen anderen Anteil berechnen muss, um somit indirekt den gewünschten Anteil des gewünschten Gases einzustellen.

Als erstes, zweites, drittes oder viertes Gas können insbesondere Sauerstoff, Kohlenstoffdioxid, Stickstoff, Neon, Xenon, Helium, Krypton oder Argon in den entsprechenden Gasvorratsbehälter aufgenommen sein. Alternativ können auch Mischungen mindestens zweier der zuvor genannten Gase in den Gasvorratsbehälter bevorratet sein, beispielsweise kann in einem der Gasvorratsbehälter eine Mischung aus 21% Sauerstoff und 79% Stickstoff, sogenannte AIR, aufgenommen sein. Ebenso kann in einem der Gasvorratsbehälter eine Mischung aus 21% Sauerstoff und 79% Helium, sogenanntes Heliox, aufgenommen sein.

Die Eingabeeinheit umfasst insbesondere einen Touchscreen, sodass die Steuereinheit auf einfache Weise gesteuert werden kann. Ferner ermöglich ein solcher Touchscreen, dass über eine einzige Einheit sowohl Daten eingegeben werden könne, insbesondere Daten über die die Zusammensetzungen des Spülgases eingegeben werden können, als auch Informationen an die Bedienperson ausgegeben werden können.

Über die Eingabeeinheit ist insbesondere ein Soll-Wert für den Volumenstrom des Spülgases einstellbar, mit dem das Spülgas den Gasbereich des Filters durchströmt. Die Steuereinheit steuert das erste Proportionalventil, das zweite Proportionalventil, das dritte Proportionalventil und/oder das Proportionalventil derart an, dass der aus den verschiedenen in den Gasvorratsbehältern aufgenommenen Gasen zusammengemischte Gasstrom des Spülgases den eingestellten Volumenstrom hat. Die Gasmischeinheit ist insbesondere derart ausgebildet, dass mit ihrer Hilfe Volumenströme zwischen 0,1 l/min und 20 l/min einstellbar sind.

Stromabwärts der Gasmischeinheit und stromaufwärts des Filters ist insbesondere eine Sensoreinheit zur Ermittlung eines Ist-Werts das Anteils des ersten Gases an dem Spülgas, eines Ist-Werts des Anteils des zweiten Gases an dem Spülgas, eines Ist-Wert des Anteils des dritten Gases an dem Spülgas und/oder eines Ist-Werts des Anteils des vierten Gases an dem Spülgas vorgesehen. Somit kann auf einfache Weise überwacht werden, ob die Zusammensetzung des Spülgases aus den einzelnen Gasen tatsächlich der gewünschten Zusammensetzung entspricht. Zusätzlich oder alternativ kann über die Sensoreinheit auch ein Ist-Wert des Volumenstroms des Spülgases ermittelbar sein, sodass auch dieser auf einfache Weise überwacht werden kann.

Die Steuereinheit vergleicht den Ist-Wert des Anteils eines der Gase vorzugsweise jeweils mit dem entsprechenden eingestellten Soll-Wert dieses Gases und steuert die Gasmischeinheit in Abhängigkeit dieses Ergebnisses dieses Vergleiches an. Insbesondere steuert die Steuereinheit die Gasmischeinheit über einen Regelkreis derart an, dass das Spülgas tatsächlich die eingestellten Anteile der einzelnen Gase umfasst. Ebenso vergleicht die Steuereinheit vorzugsweise den ermittelten Ist-Wert des Volumenstroms mit dem eingestellten Soll-Wert des Volumenstroms und steuert die Gasmischeinheit in Abhängigkeit des Ergebnisses dieses Vergleichs, insbesondere wiederum über einen Regelkreis, an.

Zusätzlich oder alternativ kann die Eingabeeinheit eine Information über mindestens einen, vorzugsweise alle, der mit Hilfe der Sensoreinheit ermittelten Ist-Werte an die Bedienperson ausgeben, sodass diese auf einfache Weise überwachen kann, ob die Zusammensetzung und/oder der Volumenstrom den eingestellten Vorgaben entspricht. Insbesondere wird über den Touchscreen eine grafische Ausgabe der einzelnen Werte, beispielsweise als Balkendiagramme, dargestellt.

Stromabwärts des Filters kann eine weitere Sensoreinheit zur Ermittlung eines weiteren Ist-Wertes des Anteils des ersten Gases an dem Spülgas, eines weiteren Ist-Wertes des Anteils zweiten Gases an dem Spülgas, eines weiteren Ist-Wertes des Anteils des dritten Gases an dem Spülgas, eines weiteren Ist-Wertes des Anteils des vierten Gases an dem Spülgas und/oder eines weiteren Ist-Wertes des Volumenstroms des Spülgases angeordnet sein. Auf diese Weise kann insbesondere ermittelt werden, ob von dem Blutstrom ein Teil mindestens eines der Gase aufgenommen wurde und wenn dies der Fall ist, welche Menge.

Über die Eingabeeinheit wird insbesondere eine Information über die mit Hilfe der weiteren Sensoreinheit ermittelten weiteren Ist-Werte an die Bedienperson ausgegeben. Insbesondere wird jeweils ein Differenzwert über den mit Hilfe der einen, stromaufwärts des Filters angeordneten Sensoreinheiten ermittelten Ist-Wertes und des entsprechenden mit Hilfe der stromabwärts des Filters angeordneten weiteren Sensoreinheit ermittelten weiteren Ist-Wertes ermittelt und dieser Differenzwert der Bedienperson angezeigt, sodass die Veränderung der Zusammensetzung des Spülgases und/oder der Strömungsgeschwindigkeit des Spülgases durch das Durchströmen des Gasbereiches des Filters auf einfache Weise ersichtlich ist.

Zusätzlich oder alternativ kann die Steuereinheit einen oder mehrere der mit Hilfe der weiteren Sensoreinheit ermittelten Ist-Werte mit den entsprechenden voreingestellten Werten vergleichen oder die Gasmischeinheit in Abhängigkeit des Ergebnisses dieses Vergleichs ansteuern.

Das erste Gas, das zweite Gas, das dritte Gas und/oder das vierte Gas umfassen insbesondere ein Medikament, sodass über den Filter auch eine Medikation des Patienten möglich ist.

Stromabwärts der Gasmischeinheit und stromaufwärts des Filters ist vorzugsweise eine Verdampfungseinheit zum Verdampfen eines volatilen Medikaments vorgesehen, mit deren Hilfe das volatile Medikament verdampft werden kann, wobei das verdampfte Medikament dem Spülgas zugeführt wird und somit zusammen mit den aus den Gasvorratsbehältern entnommenen Gasen durch den Gasbereich des Filters befördert wird, sodass das verdampfte Medikament über den Filter in den Blutstrom des Patienten gelangen kann.

Stromabwärts der Gasmischeinheit und stromaufwärts des Filters ist insbesondere ein Heizelement zum Erwärmen des Spülgases und/oder eine Befeuchtungseinheit zur Befeuchtung des Spülgases angeordnet. Durch das Erwärmen des Spülgases wird erreicht, dass die Temperaturdifferenz zwischen dem Spülgas und dem Blutstrom ausgeglichen und zumindest minimiert wird, sodass keine Auskühlung des Patienten erfolgt. Die Befeuchtungseinheit bewirkt, dass das relativ trockene Spülgas eine höhere Wasserdampfsättigung, insbesondere eine Wasserdampfsättigung von 100%, hat, sodass zum einen ein Entzug von Wasser aus dem Blutstrom und zum anderen die Ablagerung von Wasser an der Membran vermieden werden.

Stromaufwärts des Filters ist vorzugsweise ein Temperatursensor zur Ermittlung des Ist-Wertes der Temperatur des Spülgases und/oder ein Feuchtigkeitssensor zur Ermittlung der Luftfeuchtigkeit des Spülgases angeordnet, sodass die Temperatur bzw. die Feuchtigkeit des Spülgases überwacht werden kann. Insbesondere vergleicht die Steuereinheit die über diesen Sensor ermittelten Ist-Werte der Temperatur und der Luftfeuchtigkeit mit voreingestellten Werten und steuert die Heizeinheit bzw. die Befeuchtungseinheit derart an, dass diese das Spülgas derart erwärmt bzw. befeuchten, dass es die voreingestellte Temperatur bzw. die voreingestellte Luftfeuchtigkeit hat.

Zusätzlich oder alternativ können auch stromabwärts des Filters ein weiterer Temperatursensor zur Ermittlung der Temperatur des Spülgases und/oder ein weiterer Feuchtigkeitssensor zur Ermittlung der Luftfeuchtigkeit des Spülgases vorgesehen sein. Die Ist-Werte der Temperatur und/oder Luftfeuchtigkeit, die über die stromaufwärts des Filters als auch über die stromabwärts des Filters angeordneten Sensoren ermittelt wurden, werden vorzugsweise wiederum über den Touchscreen der Steuereinheit der Bedienperson angezeigt, sodass diese nicht nur die Zusammensetzung und die Strömungsgeschwindigkeit des Spülgases, sondern auch die Temperatur und Luftfeuchtigkeit vor und nach dem Durchlaufen des Filters überwachen kann.

Ferner ist es vorteilhaft, wenn eine Rückführleitung zum Rückführen des Spülgases von dem Filter in die Zuführleitung vorgesehen ist, sodass das Spülgas mehrfach verwendet werden kann. Somit muss nicht ständig neues Spülgas zugeführt werden, wodurch Kosten eingespart werden. Alternativ kann das Spülgas nach dem Durchlaufen des Filters auch recycelt und/oder entsorgt werden.

Weitere Merkmale und Vorteile der Erfindung ergeben sich aus der folgenden Beschreibung, die die Erfindung anhand von Ausführungsbeispielen in Zusammenhang mit den beigefügten Figuren näher erläutert.

Es zeigen:
- Figur 1: eine schematische Darstellung einer Anordnung zum Entfernen von Kohlenstoffdioxid aus einem exkorporalen Blutstrom und/oder zum Anreichern des exkorporalen Blutstroms mit Sauerstoff gemäß einer ersten Ausführungsform; und
- Figur 2: eine schematische Darstellung einer Anordnung zum Entfernen von Kohlenstoffdioxid aus einem exkorporalen Blutstrom und/oder zum Anreichern des exkorporalen Blutstroms mit Sauerstoff gemäß einer zweiten Ausführungsform.

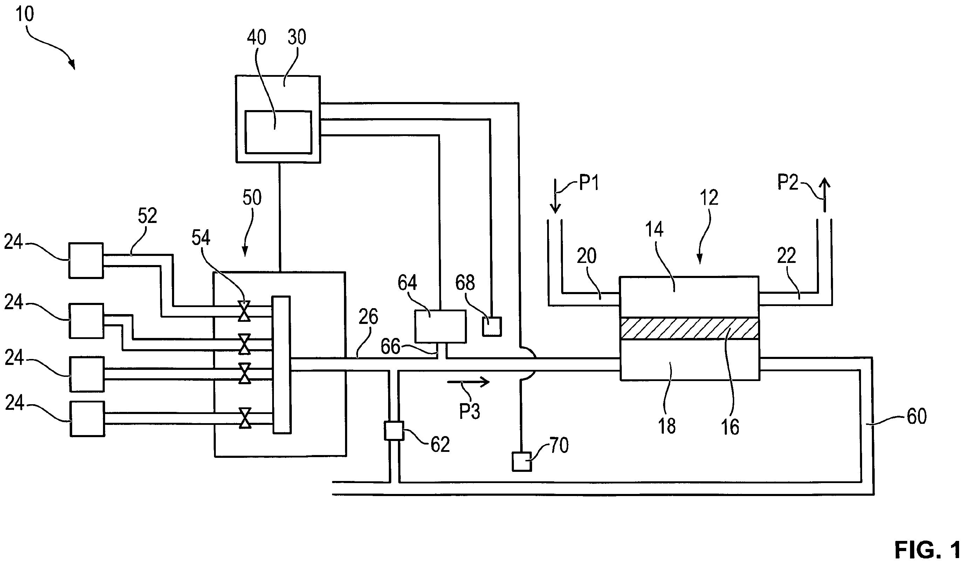

In Figur 1 ist eine schematische Darstellung einer Anordnung 10 zum Entfernen von Kohlenstoffdioxid aus einem exkorporalen Blutstrom eines Patienten und zum Anreichern des exkorporalen Blutstroms mit Sauerstoff gezeigt. Die Anordnung 10 umfasst einen als Oxygenerator 12 ausgebildeten Filter, der im Blutbereich 14 und einen über eine Membran 16 von diesem Blutbereich 14 getrennten Gasbereich 18 aufweist. Durch den Blutbereich 14 wird der exkorporale Blutstrom entsprechend der Pfeile P1 und P2 hindurch gefördert, wozu eine Zuführleitung 20 und eine Abflussleitung 22 vorgesehen sind. Es kann auch ein Blutpumpe vorgesehen sein, mit deren Hilfe der Blutstrom durch den Blutbereich 14 erzeugt wird.

Durch den Gasbereich 18 wird ein Spülgas gefördert, was durch den Pfeil P3 angedeutet ist. Ferner umfasst die Anordnung 20 eine über eine Steuereinheit 30 elektronisch gesteuerte Gasmischeinheit 50, die auch als Gasmischbank, bezeichnet wird. Die Anordnung 10 hat vier Gasvorratsbehälter 24, die jeweils über eine Leitung 52 mit der Gasmischeinheit 50 verbunden sind. In den Gasvorratsbehältern 24 ist jeweils ein Gas oder ein Gemisch verschiedener Gase aufgenommen.

Die Gasmischeinheit 50 weist vier Proportionalventile auf, von denen eines beispielhaft mit dem Bezugszeichen 54 bezeichnet ist. Jeweils eines der Proportionalventile 54 dient zum Regeln des Volumenstromes, der einem der Gasvorratsbehälter 24 entnommen wird. Die über die Proportionalventile 54 den einzelnen Gasvorratsbehältern 24 entnommenen Gase werden zu dem Spülgas zusammengemischt und über eine Zuführleitung 26 dem Gasbereich 18 des Oxygenerators 12 zugeführt.

Aufgrund eines zwischen einzelnen Komponenten des Spülgases und der entsprechenden Komponente in dem Blutstrom bestehenden Paritaldruckgradienten bzw. einen bestehenden Konzentrationsunterschied wird diese Komponente durch Membran 16 hindurch von dem Gasbereich 18 in den Blutbereich 14 oder umgekehrt befördert. Insbesondere wird auf diese Weise Sauerstoff von dem Gasbereich 18 in den Blutbereich 14 befördert, sodass der Blutstrom mit Sauerstoff angereichert wird. Umgekehrt wird Kohlenstoffdioxid von dem Blutbereich 14 in den Gasbereich 18 durch die Membranen 16 hindurch befördert, sodass Kohlenstoffdioxid aus dem Blutstrom entfernt wird. Die Transferleistung des Sauerstoffes bzw. des Kohlenstoffdioxid, d. h. die Menge an Kohlenstoffdioxid bzw. Sauerstoff, die pro Zeiteinheit dem Blutstrom zugeführt bzw. entnommen wird, hängt u.a. von der Strömungsgeschwindigkeit des Blutstroms, des Volumenstroms des Spülgases und der Zusammensetzung des Spülgases ab.

Insbesondere kann über die Zusammensetzung des Spülgases auch gesteuert werden, ob lediglich Sauerstoff zugeführt wird, lediglich Kohlenstoffdioxid entnommen wird oder beides zeitglich erfolgt. Daher ist es wichtig, dass die Zusammensetzung des Spülgases und der Volumenstrom des Spülgases möglichst genau eingestellt werden können, was durch die zuvor beschriebene elektronisch gesteuerte Gasmischeinheit 50 einfach möglich ist.

In den Gasvorratsbehältern 24 sind als Gase insbesondere Stickstoff, Kohlenstoffdioxid, Sauerstoff, Helium, Xenon, Neon, Argon, Krypton eine Mischung aus 21% Sauerstoff und 79% Stickstoff, eine Mischung aus 21% Sauerstoff und 79% Helium oder weitere medizinische Gase oder Gasmischungen aufgenommen. Über die Proportionalventile 54 kann der Anteil der den Gasvorratsbehältern 24 entnommen Gase an dem Spülgas und somit auch der Volumenstrom des Spülgases automatisch und genau eingestellt werden. Die Steuereinheit 30 weist insbesondere ein Touchscreen 40 auf, über den eine Bedienperson der Anordnung 10 auf einfache Weise die gewünschte Zusammensetzung des Spülgases, d. h. die Anteile der einzelnen Gase, einstellen kann. Ferner kann die Bedienperson über die Steuereinheit 30 den Volumenstrom des Spülgases einstellen. Die Steuereinheit 30 ermittelt in Abhängigkeit der von der Bedienperson eingestellten Werte insbesondere Stellgrößen für die einzelnen Proportionalventile 54 und steuert die Gasmischeinheit 50 entsprechend an. Somit kann die Bedienperson auf einfache Weise direkt die Anteile eingeben und muss nicht aufwendig die Stellgrößen der einzelnen Proportionalventile 54 berechnen oder durch Probieren herausfinden, wie dies beispielsweise bei mechanischen Gasblendern der Fall ist.

Bei einer alternativen Ausführungsform der Erfindung können auch mehr als vier Gasvorratsbehälter 24, beispielsweise fünf Gasvorratsbehälter 24, oder weniger als vier Gasvorratsbehälter 24, beispielsweise zwei Gasvorratsbehälter 24, vorgesehen sein.

Nach dem Durchlaufen des Gasbereiches 18 des Oxygenerators 12 wird das Spülgas über eine Rückführleitung 60 wieder zurück zu der Zuführleitung 26 befördert, sodass das Spülgas mehrfach zum Durchströmen des Gasbereiches 18 verwendet werden kann und somit nicht ständig neues Gas den Gasvorratsbehälter 24 entnommen werden muss. Insbesondere wird das rückgeführte Gas mit neu den Gasvorratsbehältern 24 entnommen Gas gemischt bevor es wieder dem Oxygenerator 12 zugeführt wird. Die Rückführleitung 60 ist vorzugsweise an der Gasmischeinheit 50 angeschlossen, sodass das zurückgeführte Spülgas über die Gasmischeinheit 50 mit eventuell den Vorratsbehältern 24 entnommenen Gasen gemischt werden kann. Alternativ kann die Verbindung der Rückführleitung 60 mit der Zuführleitung 26 auch stromabwärts der Gasmischeinheit 50 erfolgen.

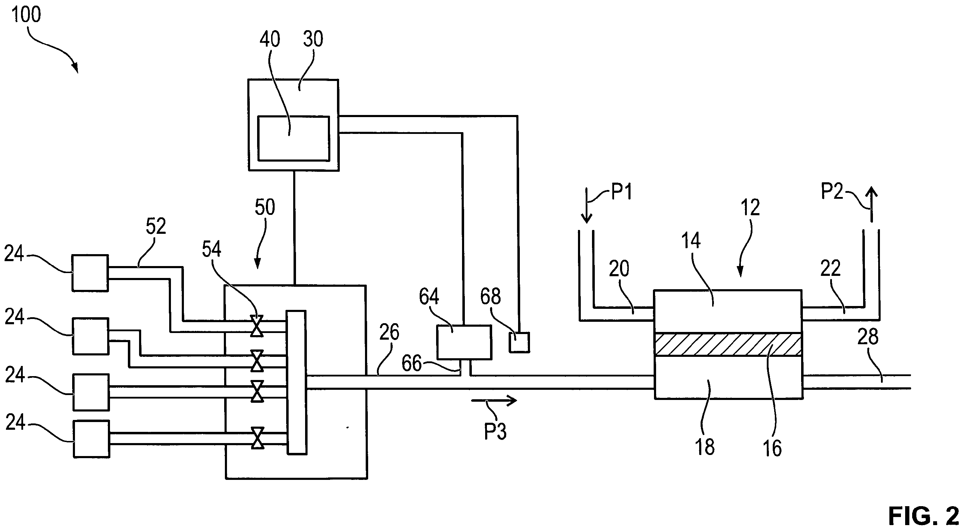

Bei einer zweiten Ausführungsform, die in Figur 2 gezeigt ist, ist anstelle einer Rückführleitung 60 nur eine Abführleitung 28 der Anordnung 100 vorgesehen, über die das Spülgas nach dem Durchströmen des Gasbereiches 18 einer Recyclings- oder Entsorgungseinheit zugeführt wird.

Im Bereich der Rückführleitung 60 ist insbesondere ein Ventilator 62 angeordnet, mit dessen Hilfe die Strömung des Spülgases aufrechterhalten wird. Bei einer alternativen Ausführungsform kann der Gasstrom des Spülgases auch lediglich durch den Druck, mit dem die Gase in den Gasvorratsbehältern 24 aufgenommen sind, erzeugt werden.

Darüber hinaus ist eine Verdampfungseinheit 64 vorgesehen, mit deren Hilfe volatile Medikamente verdampft werden können. Das verdampfte Medikament wird über eine Verbindungsleitung 66 der Zuführleitung 26 zugeführt, sodass auch das verdampfte Medikament über den Oxygenerator 12 an den Blutstrom übertragen und somit dem Patienten verabreicht werden kann. Die Verdampfungseinheit 64 wird insbesondere ebenfalls über die Steuereinheit 30 gesteuert.

Ferner umfasst die Anordnung 10 zwei Sensoreinheiten 68, 70, wobei eine der Sensoreinheiten 68 stromaufwärts des Oxygenerators 12 im Bereich der Zuführleitung 26 und die andere Sensoreinheit 70 stromabwärts des Oxygenerators 12 im Bereich der Rückführleitung 60 bzw. der Abführleitung 28 angeordnet ist. Mit Hilfe der Sensoreinheiten 68, 70 kann insbesondere die Zusammensetzung des Spülgases ermittelt werden, d.h. es wird ermittelt, wie hoch der Ist-Wert der Anteile der einzelnen Gase an dem Spülgas ist. Die ermittelten Ist-Werte werden insbesondere mit den von der Bedienperson von der Steuereinheit 30 voreingestellten Soll-Werte verglichen, wobei die Steuereinheit 30 in Abhängigkeit des Ergebnisses dieses Vergleiches die Gasmischeinheit 50 in Form eines Regelkreises derart ansteuert, dass der tatsächliche Anteil des jeweiligen Gases dem voreingestellten Soll-Wert entspricht.

Zusätzlich kann über die Sensoreinheiten 68, 70 auch jeweils die Strömungsgeschwindigkeit des Spülgases ermittelt werden, wobei die Strömungseinheit vorzugsweise wiederum den ermittelten Ist-Wert der Strömungsgeschwindigkeit mit dem voreingestellten Soll-Wert vergleicht und die einzelnen Proportionalventile 54 derart ansteuert, dass die tatsächliche Strömungsgeschwindigkeit dem Soll-Wert entspricht.

Zusätzlich oder alternativ können die über die Sensoreinheiten 68, 70 ermittelten Ist-Werte auch der Bedienperson über den Touchscreen 40 angezeigt werden, sodass diese auf einfache Weise die ordnungsgemäße Funktion der Anordnung 10 überwachen kann. Insbesondere wird ein Differenzwert aus den stromaufwärts und stromabwärts des Oxygenerators 12 ermittelten Ist-Werten der jeweiligen Anteile der Gase der Bedienperson, vorzugsweise grafisch, angezeigt, sodass diese auf einfache Weise überwachen kann, wie viel Kohlenstoffdioxid dem Blutstrom entnommen bzw. wie viel Sauerstoff dem Blutstrom zugeführt wurde.

Darüber hinaus können die Sensoreinheit 68, 70 auch Temperatursensoren zur Ermittlung der Temperatur des Spülgases und/oder Feuchtigkeitssensoren zur Ermittlung der Luftfeuchtigkeit des Spülgases umfassen. Ebenso können stromaufwärts des Oxygenerators eine Heizeinheit zum Erwärmen des Spülgases und/oder eine Befeuchtungseinheit zur Erhöhung der Luftfeuchtigkeit des Spülgases vorgesehen sein.

### Bezugszeichenliste

- 10, 100: Anordnung
- 12: Oxygenerator
- 14: Blutbereich
- 16: Membran
- 18: Gasbereich
- 20, 22: Leitung
- 24: Gasvorratsbehälter
- 26: Zuführleitung
- 28: Abführleitung
- 30: Steuereinheit
- 40: Touchscreen
- 50: Gasmischeinheit
- 52: Leitung
- 54: Proportionalventil
- 60: Rückführleitung
- 62: Ventilator
- 64: Verdampfungseinheit
- 66: Leitung
- 68, 70: Sensoreinheit
- P1, P2, P3: Richtung

## Patentansprüche

1. Anordnung zum Entfernen von Kohlenstoffdioxid aus einem exkorporalen Blutstrom und/oder zum Anreichern des exkorporalen Blutstroms mit Sauerstoff,
mit einem Filter (12), der eine Membran (16) umfasst, die einen Blutbereich (14), durch den der exkorporale Blutstrom geführt ist, von einem Gasbereich (18), durch den ein Gasstrom eines Spülgases geführt ist, trennt,
einem ersten Gasvorratsbehälter (24), in dem ein erstes Gas aufgenommen ist, und mindestens einem zweiten Gasvorratsbehälter (24), in dem ein von dem ersten Gas verschiedenes zweites Gas aufgenommen ist,
einer Gasmischeinheit (50) zum Mischen des ersten Gases und des zweiten Gases zu dem Spülgas, und
mit einer Zuführleitung (26) zum Zuführen des Spülgases zu dem Gasbereich (18) des Filters,
**dadurch gekennzeichnet, dass** eine elektronische Steuereinheit (30) zum Steuern der Gasmischeinheit (50) vorgesehen ist,
die Steuereinheit (30) eine Eingabeeinheit (40) umfasst, mit deren Hilfe der Anteil des ersten Gases an dem Spülgas von einer Bedienperson einstellbar ist, und
dass die Steuereinheit (30) die Gasmischeinheit (50) derart ansteuert, dass das Spülgas den eingestellten Anteil des ersten Gases aufweist,
dass die Gasmischeinheit (50) ein erstes Proportionalventil (54) zum Einstellen des aus dem ersten Gasvorratsbehälter (24) entnommenen Volumenstroms des ersten Gases und ein zweites Proportionalventil (54) zum Einstellen des dem zweiten Gasvorratsbehälter (24) entnommenen Volumenstroms des zweiten Gases umfasst, und
dass die Steuereinheit (30) zum Einstellen des über die Eingabeeinheit (40) eingestellten Anteils des ersten Gases das erste Proportionalventil (54) und/oder das zweite Proportionalventil (54) ansteuert, und
dass stromabwärts der Gasmischeinheit (50) und stromaufwärts des Filters (12) eine Heizeinheit zum Erwärmen des Spülgases angeordnet ist,
dass stromaufwärts des Filters (12) ein Temperatursensor zur Ermittlung eines Ist-Wertes der Temperatur des Spülgases angeordnet ist,
dass stromabwärts des Filters (12) ein weiterer Temperatursensor zur Ermittlung der Temperatur des Spülgases angeordnet ist, und
dass die Steuereinheit (30) die Heizeinheit derart steuert, dass die Heizeinheit das Spülgas abhängig von der mit Hilfe des ersten und/oder zweiten Temperatursensors ermittelten Temperatur erwärmt.

2. Anordnung (10, 100) nach Anspruch 1, **dadurch gekennzeichnet, dass** die Steuereinheit (30) in Abhängigkeit des eingestellten Anteils des ersten Gases einen ersten Stellwert für das erste Proportionalventil (54) und/oder einen zweiten Stellwert für das zweite Proportionalventil (54) ermittelt, und dass die Steuereinheit (30) am ersten Proportionalventil (54) den ersten Stellwert und/oder am zweiten Proportionalventil (54) den zweiten Stellwert einstellt.

3. Anordnung (10, 100) nach einem der vorhergehenden Ansprüche, **dadurch gekennzeichnet, dass** ein dritter Gasvorratsbehälter (24), in dem ein drittes Gas aufgenommen ist, und mindestens ein vierter Gasvorratsbehälter (24), in dem ein viertes Gas aufgenommen ist, vorgesehen sind, dass die Gasmischeinheit (50) aus dem ersten Gas, dem zweiten Gas, dem dritten Gas und/oder dem vierten Gas das Spülgas mischt, dass über die Eingabeeinheit (40) der Anteil mindestens dreier Gase einstellbar ist, und dass die Steuereinheit (30) die Gasmischeinheit (50) derart ansteuert, dass das Spülgas die eingestellten Anteile der Gase aufweist.

4. Anordnung (10, 100) nach Anspruch 3, **dadurch gekennzeichnet, dass** die Gasmischeinheit (50) ein drittes Proportionalventil (54) zum Einstellen des aus dem dritten Gasvorratsbehälter (24) entnommenen Volumenstroms des dritten Gases und/oder ein viertes Proportionalventil (54) zum Einstellen des dem vierten Gasvorratsbehälter (24) entnommenen Volumenstroms des vierten Gases umfasst, und dass die Steuereinheit (30) zum Einstellen des eingestellten Anteils des Gases das dritte Proportionalventil (54) und/oder das vierte Proportionalventil (54) ansteuert.

5. Anordnung (10, 100) nach Anspruch 4, **dadurch gekennzeichnet, dass** die Steuereinheit (30) in Abhängigkeit des eingestellten Anteils des dritten Gases einen dritten Stellwert für das dritte Proportionalventil (54) und/oder in Abhängigkeit des eingestellten Anteils des vierten Gases einen vierten Stellwert für das vierte Proportionalventil (54) ermittelt, und dass die Steuereinheit (30) am dritten Proportionalventil (54) den dritten Stellwert und/oder am vierten Proportionalventil (54) den vierten Stellwert einstellt.

6. Anordnung (10, 100) nach einem der Ansprüche 3 bis 5, **dadurch gekennzeichnet, dass** über die Eingabeeinheit (40) der Anteil aller vier Gase einstellbar ist.

7. Anordnung (10, 100) nach einem der vorhergehenden Ansprüche, **dadurch gekennzeichnet, dass** die Eingabeeinheit (40) einen Touchscreen umfasst.

8. Anordnung (10, 100) nach einem der vorhergehenden Ansprüche, **dadurch gekennzeichnet, dass** über die Eingabeeinheit (40) ein Volumenstrom des Spülgases, insbesondere im Bereich zwischen 0,1 l/min und 20 l/min, einstellbar ist, und dass die Steuereinheit (30) das erste Proportionalventil (54), das zweite Proportionalventil (54), das dritte Proportionalventil (54) und/oder das vierte Proportionalventil (54) derart ansteuert, dass das Spülgas den eingestellten Volumenstrom hat.

9. Anordnung (10, 100) nach einem der vorhergehenden Ansprüche, **dadurch gekennzeichnet, dass** die Eingabeeinheit (40) eine Information über die mit Hilfe der Sensoreinheit (68) ermittelten Ist-Werte an die Bedienperson ausgibt.

10. Anordnung (10, 100) nach einem der vorhergehenden Ansprüche, **dadurch gekennzeichnet, dass** stromabwärts des Filters (12) eine weitere Sensoreinheit (70) zur Ermittlung eines weiteren Ist-Werts des Anteils des ersten Gases an dem Spülgas, eines weiteren Ist-Werts des Anteils des zweiten Gases an dem Spülgas, eines weiteren Ist-Werts des Anteils des dritten Gases an dem Spülgas, eines weiteren Ist-Werts des Anteils des vierten Gases an dem Spülgas und/oder eines weiteren Ist-Werts des Volumenstroms des Spülgases angeordnet ist, und dass die Eingabeeinheit (40) eine Information über die mit Hilfe der weiteren Sensoreinheit (70) ermittelten weiteren Ist-Werte an die Bedienperson ausgibt und/oder die Steuereinheit (30) den weiteren Ist-Wert des Anteils des ersten Gases, den weiteren Ist-Wert des Anteils des zweiten Gases, den weiteren Ist-Wert des Anteils des dritten Gases, den weiteren Ist-Wert des Anteils des vierten Gases und/oder den weiteren Ist-Wert des Volumenstroms des Spülgases mit dem jeweils entsprechenden eingestellten Wert vergleicht und die Gasmischeinheit (50) in Abhängigkeit des Ergebnisses dieses Vergleichs ansteuert.

11. Anordnung (10, 100) nach Anspruch 10, **dadurch gekennzeichnet, dass** die Steuereinheit (30) jeweils einen Differenzwert zwischen dem Ist-Wert und dem entsprechenden weiteren Ist-Wert ermittelt und Informationen über die ermittelten Differenzwerte, insbesondere in Form einer Graphik, über die Eingabeeinheit (40) an die Bedienperson ausgibt.

12. Anordnung (10, 100) nach einem der vorhergehenden Ansprüche, **dadurch gekennzeichnet, dass** stromabwärts der Gasmischeinheit (50) eine Befeuchtungseinheit zur Befeuchtung des Spülgases angeordnet ist.

13. Anordnung (10, 100) nach einem der vorhergehenden Ansprüche, **dadurch gekennzeichnet, dass** stromaufwärts des Filters (12) ein Feuchtigkeitssensor zur Ermittlung der Luftfeuchtigkeit des Spülgases angeordnet ist.

14. Anordnung (10, 100) nach einem der vorhergehenden Ansprüche, **dadurch gekennzeichnet, dass** stromabwärts des Filters (12) ein weiterer Feuchtigkeitssensor zur Ermittlung der Luftfeuchtigkeit des Spülgases vorgesehen ist.

## Claims

1. Arrangement for removing carbon dioxide from an extracorporeal blood stream and/or for enriching the extracorporeal blood stream with oxygen,
having a filter (12) comprising a membrane (16) that separates a blood region (14), through which the extracorporeal blood stream is guided, from a gas region (18), through which a gas stream of a purge gas is guided,
a first gas storage tank (24) in which a first gas is received, and at least a second gas storage tank (24) in which a second gas, different from the first gas, is received,
a gas mixer unit (50) for mixing the first gas and the second gas to give the purge gas, and
having a supply line (26) for supplying the purge gas to the gas region (18) of the filter,
**characterized in that** an electronic control unit (30) for controlling the gas mixer unit (50) is provided,
the control unit (30) comprises an input unit (40), with the aid of which the fraction of the first gas in the purge gas is adjustable by an operator, and
**in that** the control unit (30) controls the gas mixer unit (50) in such a way that the purge gas has the adjusted fraction of the first gas,
**in that** the gas mixer unit (50) comprises a first proportional valve (54) for adjusting the volumetric flow of the first gas withdrawn from the first gas storage tank (24), and a second proportional valve (54) for adjusting the volumetric flow of the second gas withdrawn from the second gas storage tank (24), and
**in that** the control unit (30) controls the first proportional valve (54) and/or the second proportional valve (54) in order to adjust the fraction of the first gas adjusted via the input unit (40), and
**in that** a heating unit for heating the purge gas is arranged downstream from the gas mixer unit (50) and upstream from the filter (12),
**in that** a temperature sensor for determining an actual value of the temperature of the purge gas is arranged upstream from the filter (12),
**in that** a further temperature sensor for determining the temperature of the purge gas is arranged downstream from the filter (12), and
**in that** the control unit (30) controls the heating unit in such a way that the heating unit heats the purge gas according to the temperature determined with the aid of the first and/or second temperature sensor.

2. Arrangement (10, 100) according to Claim 1, **characterized in that** the control unit (30) determines, as a function of the adjusted fraction of the first gas, a first setpoint for the first proportional valve (54) and/or a second setpoint for the second proportional valve (54), and **in that** the control unit (30) adjusts the first setpoint at the first proportional valve (54) and/or the second setpoint at the second proportional valve (54).

3. Arrangement (10, 100) according to either of the preceding claims, **characterized in that** a third gas storage tank (24), in which a third gas is received, and at least a fourth gas storage tank (24), in which a fourth gas is received, are provided, **in that** the gas mixer unit (50) mixes the purge gas from the first gas, the second gas, the third gas and/or the fourth gas, **in that** the fraction of at least three gases is adjustable via the input unit (40), and **in that** the control unit (30) controls the gas mixer unit (50) in such a way that the purge gas has the adjusted fractions of the gases.

4. Arrangement (10, 100) according to Claim 3, **characterized in that** the gas mixer unit (50) comprises a third proportional valve (54) for adjusting the volumetric flow of the third gas withdrawn from the third gas storage tank (24) and/or a fourth proportional valve (54) for adjusting the volumetric flow of the fourth gas withdrawn from the fourth gas storage tank (24), and **in that** the control unit (30) controls the third proportional valve (54) and/or the fourth proportional valve (54) in order to adjust the adjusted fraction of the gas.

5. Arrangement (10, 100) according to Claim 4, **characterized in that** the control unit (30) determines a third setpoint for the third proportional valve (54) as a function of the adjusted fraction of the third gas and/or determines a fourth setpoint for the fourth proportional valve (54) as a function of the adjusted fraction of the fourth gas, and **in that** the control unit (30) adjusts the third setpoint at the third proportional valve (54) and/or the fourth setpoint at the fourth proportional valve (54).

6. Arrangement (10, 100) according to one of Claims 3 to 5, **characterized in that** the fraction of all four gases is adjustable via the input unit (40).

7. Arrangement (10, 100) according to one of the preceding claims, **characterized in that** the input unit (40) comprises a touchscreen.

8. Arrangement (10, 100) according to one of the preceding claims, **characterized in that** a volumetric flow of the purge gas is adjustable via the input unit (40), in particular in the range between 0.1 l/min and 20 l/min, and **in that** the control unit (30) controls the first proportional valve (54), the second proportional valve (54), the third proportional valve (54) and/or the fourth proportional valve (54) in such a way that the purge gas has the adjusted volumetric flow.

9. Arrangement (10, 100) according to one of the preceding claims, **characterized in that** the input unit (40) outputs, to the operator, an item of information concerning the actual values determined with the aid of the sensor unit (68).

10. Arrangement (10, 100) according to one of the preceding claims, **characterized in that**, downstream from the filter (12), a further sensor unit (70) is arranged for determining a further actual value of the fraction of the first gas in the purge gas, a further actual value of the fraction of the second gas in the purge gas, a further actual value of the fraction of the third gas in the purge gas, a further actual value of the fraction of the fourth gas in the purge gas and/or a further actual value of the volumetric flow of the purge gas, and **in that** the input unit (40) outputs, to the operator, an item of information concerning the further actual values determined with the aid of the further sensor unit (70), and/or the control unit (30) compares the further actual value of the fraction of the first gas, the further actual value of the fraction of the second gas, the further actual value of the fraction of the third gas, the further actual value of the fraction of the fourth gas and/or the further actual value of the volumetric flow of the purge gas with the respectively corresponding adjusted value and controls the gas mixer unit (50) as a function of the result of this comparison.

11. Arrangement (10, 100) according to Claim 10, **characterized in that** the control unit (30) determines respectively a difference value between the actual value and the corresponding further actual value and outputs, to the operator, information concerning the determined difference values, in particular in the form of a graph, via the input unit (40).

12. Arrangement (10, 100) according to one of the preceding claims, **characterized in that** a humidifying unit for humidifying the purge gas is arranged downstream from the gas mixer unit (50).

13. Arrangement (10, 100) according to one of the preceding claims, **characterized in that** a humidity sensor for determining the air humidity of the purge gas is arranged upstream from the filter (12).

14. Arrangement (10, 100) according to one of the preceding claims, **characterized in that** a further humidity sensor for determining the air humidity of the purge gas is arranged downstream from the filter (12).

## Revendications

1. Arrangement pour supprimer le dioxyde de carbone d'un flux sanguin extracorporel et/ou pour enrichir le flux sanguin extracorporel avec de l'oxygène,
comprenant un filtre (12), lequel comporte une membrane (16) qui sépare une zone de sang (14), à travers laquelle passe le flux sanguin extracorporel, d'une zone de gaz (18), à travers laquelle passe un courant de gaz d'un gaz de purge,
un premier réservoir de gaz (24), dans lequel est accueilli un premier gaz, et au moins un deuxième réservoir de gaz (24), dans lequel est accueilli un deuxième gaz différent du premier gaz,
une unité de mélange de gaz (50) destinée à mélanger le premier gaz et le deuxième gaz pour obtenir le gaz de purge, et
comprenant une conduite d'acheminement (26) servant à acheminer le gaz de purge vers la zone de gaz (18) du filtre,
**caractérisé en ce qu'**une unité de commande électronique (30) destinée à commander l'unité de mélange de gaz (50) est présente,
l'unité de commande (30) comporte une unité de saisie (40) à l'aide de laquelle la proportion du premier gaz dans le gaz de purge peut être réglée par un opérateur, et
**en ce que** l'unité de commande (30) commande l'unité de mélange de gaz (50) de telle sorte que le gaz de purge présente la proportion réglée du premier gaz,
**en ce que** l'unité de mélange de gaz (50) comporte une première vanne proportionnelle (54) destinée à régler le débit volumique du premier gaz prélevé du premier réservoir de gaz (24) et une deuxième vanne proportionnelle (54) destinée à régler le débit volumique du deuxième gaz prélevé du deuxième réservoir de gaz (24), et
**en ce que** l'unité de commande (30) commande la première vanne proportionnelle (54) et/ou la deuxième vanne proportionnelle (54) en vue de régler la proportion du premier gaz réglée par le biais de l'unité de saisie (40), et
**en ce qu'**une unité de chauffage destinée au chauffage du gaz de purge est disposée en aval de l'unité de mélange de gaz (50) et en amont du filtre (12),
**en ce qu'**une sonde de température destinée à déterminer une valeur réelle de la température du gaz de purge est disposée en amont du filtre (12),
**en ce qu'**une sonde de température supplémentaire destinée à déterminer la température du gaz de purge est disposée en aval du filtre (12), et
**en ce que** l'unité de commande (30) commande l'unité de chauffage de telle sorte que l'unité de chauffage chauffe le gaz de purge en fonction de la température déterminée à l'aide de la première et/ou de la deuxième sonde de température.

2. Arrangement (10, 100) selon la revendication 1, **caractérisé en ce que** l'unité de commande (30) détermine une première valeur de commande pour la première vanne proportionnelle (54) et/ou une deuxième valeur de commande pour la deuxième vanne proportionnelle (54) en fonction de la proportion réglée du premier gaz et **en ce que** l'unité de commande (30) règle la première valeur de commande sur la première vanne proportionnelle (54) et/ou la deuxième valeur de commande sur la deuxième vanne proportionnelle (54).

3. Arrangement (10, 100) selon l'une des revendications précédentes, **caractérisé en ce qu'**il existe un troisième réservoir de gaz (24), dans lequel est accueilli un troisième gaz, et au moins un quatrième réservoir de gaz (24), dans lequel est accueilli un quatrième gaz, **en ce que** l'unité de mélange de gaz (50) mélange le gaz de purge à partir du premier gaz, du deuxième gaz, du troisième gaz et/ou du quatrième gaz, **en ce que** la proportion d'au moins trois gaz peut être réglée par le biais de l'unité de saisie (40) et **en ce que** l'unité de commande (30) commande l'unité de mélange de gaz (50) de telle sorte que le gaz de purge présente les proportions réglées des gaz.

4. Arrangement (10, 100) selon la revendication 3, **caractérisé en ce que** l'unité de mélange de gaz (50) comporte une troisième vanne proportionnelle (54) destinée à régler le débit volumique du troisième gaz prélevé du troisième réservoir de gaz (24) et/ou une quatrième vanne proportionnelle (54) destinée à régler le débit volumique du quatrième gaz prélevé du quatrième réservoir de gaz (24), et **en ce que** l'unité de commande (30) commande la troisième vanne proportionnelle (54) et/ou la quatrième vanne proportionnelle (54) en vue de régler la proportion réglée du gaz.

5. Arrangement (10, 100) selon la revendication 4, **caractérisé en ce que** l'unité de commande (30) détermine une troisième valeur de commande pour la troisième vanne proportionnelle (54) en fonction de la proportion réglée du troisième gaz et/ou une quatrième valeur de commande pour la quatrième vanne proportionnelle (54) en fonction de la proportion réglée du quatrième gaz, et **en ce que** l'unité de commande (30) règle la troisième valeur de réglage sur la troisième vanne proportionnelle (54) et/ou la quatrième valeur de commande sur la quatrième vanne proportionnelle (54).

6. Arrangement (10, 100) selon l'une des revendications 3 à 5, **caractérisé en ce que** la proportion de tous les quatre gaz est réglable par le biais de l'unité de saisie (40).

7. Arrangement (10, 100) selon l'une des revendications précédentes, **caractérisé en ce que** l'unité de saisie (40) comporte un écran tactile.

8. Arrangement (10, 100) selon l'une des revendications précédentes, **caractérisé en ce qu'**un débit volumique du gaz de purge peut être réglé par le biais de l'unité de saisie (40), notamment dans la plage entre 0,1 l/min et 20 l/min, et **en ce que** l'unité de commande (30) commande la première vanne proportionnelle (54), la deuxième vanne proportionnelle (54), la troisième vanne proportionnelle (54) et/ou la quatrième vanne proportionnelle (54) de telle sorte que le gaz de purge présente le débit volumique réglé.

9. Arrangement (10, 100) selon l'une des revendications précédentes, **caractérisé en ce que** l'unité de saisie (40) délivre à l'opérateur une information à propos des valeurs réelles déterminées à l'aide de l'unité de détection (68).

10. Arrangement (10, 100) selon l'une des revendications précédentes, **caractérisé en ce qu'**en amont du filtre (12) est disposée une unité de détection (70) supplémentaire destinée à déterminer une valeur réelle supplémentaire de la proportion du premier gaz dans le gaz de purge, une valeur réelle supplémentaire de la proportion du deuxième gaz dans le gaz de purge, une valeur réelle supplémentaire de la proportion du troisième gaz dans le gaz de purge, une valeur réelle supplémentaire de la proportion du quatrième gaz dans le gaz de purge et/ou une valeur réelle supplémentaire du débit volumique du gaz de purge, et **en ce que** l'unité de saisie (40) délivre à l'opérateur une information à propos des valeurs réelles supplémentaires déterminées à l'aide de l'unité de détection (70) supplémentaire et/ou l'unité de commande (30) compare la valeur réelle supplémentaire de la proportion du premier gaz, la valeur réelle supplémentaire de la proportion du deuxième gaz, la valeur réelle supplémentaire de la proportion du troisième gaz, la valeur réelle supplémentaire de la proportion du quatrième gaz et/ou la valeur réelle supplémentaire du débit volumique du gaz de purge avec la valeur réglée correspondante respective et commande l'unité de mélange de gaz (50) en fonction du résultat de cette comparaison.

11. Arrangement (10, 100) selon la revendication 10, **caractérisé en ce que** l'unité de commande (30) détermine respectivement une valeur différentielle entre la valeur réelle et la valeur réelle supplémentaire correspondante et délivre à l'opérateur, par le biais de l'unité de saisie (40), des informations à propos des valeurs différentielles déterminées, notamment sous la forme d'un graphique.

12. Arrangement (10, 100) selon l'une des revendications précédentes, **caractérisé en ce qu'**une unité d'humidification destinée à humidifier le gaz de purge est disposée en aval de l'unité de mélange de gaz (50) .

13. Arrangement (10, 100) selon l'une des revendications précédentes, **caractérisé en ce qu'**un capteur d'humidité destiné à déterminer l'humidité de l'air du gaz de purge est disposé en amont du filtre (12) .

14. Arrangement (10, 100) selon l'une des revendications précédentes, **caractérisé en ce qu'**un capteur d'humidité supplémentaire destiné à déterminer l'humidité de l'air du gaz de purge se trouve en aval du filtre (12).
